# EUROPEAN PATENT SPECIFICATION

(11) **EP 3 522 076 B1**
(45) Date of publication and mention of the grant of the patent: **02.08.2023**
(21) Application number: 17850895.8
(22) Date of filing: 12.09.2017
(51) Int. Cl.: G06K 17/00, A01N 1/02, A61D 19/02, C12M 1/24, G06K 7/00, G06K 7/10, G06K 19/077, A61B 17/425, G06Q 10/08

(54) **INDIVIDUAL MANAGEMENT SYSTEM**
INDIVIDUELLES MANAGEMENTSYSTEM
SYSTÈME DE GESTION INDIVIDUELLE

(30) Priority: 13.09.2016 JP 2016179050
(43) Date of publication of application: 07.08.2019
(73) Proprietor: 77 KC Co., Ltd., Yamato-shi, Kanagawa 242-0007 (JP)
(72) Inventor: KOMATSU, Hirohide, Yokohama-shi, Kanagawa 227-0066 (JP); SAWA, Tsutomu, Fujisawa-shi, Kanagawa 252-0804 (JP)
(74) Representative: Weickmann & Weickmann PartmbB
(86) International application number: PCT/JP2017/032945
(87) International publication number: WO 2018/051994

(56) References cited:
- EP-A2- 2 779 020
- WO-A1-2007/077996
- JP-A- 2006 004 299
- JP-A- 2007 066 011
- JP-A- 2007 066 011
- JP-A- 2014 212 724
- JP-A- 2014 212 724
- JP-A- 2015 109 507
- JP-A- 2015 222 539
- JP-A- 2015 222 539
- US-A1- 2006 138 232
- US-A1- 2016 026 911

## Description

### Technical Field

The present invention relates to an individual management system which is suitable for managing individually reproductive cells such as sperms, ova or fertilized eggs of patients and such as sperms, ova or fertilized eggs of animals which are cryopreserved.

### Background Art

In recent years, as for infertility treatments, treatments such as in-vitro fertilization or microinsemination are executed widely. The number of in-vitro fertilization executed are increasing suddenly from about 2002. The result number of in-vitro fertilization executed was about 370,000 in 2013 and it is expected that the number of in-vitro fertilization executed will be more than 700,000 in 2020. Babies were born by in-vitro fertilization in the ratio of one out of about 27 babies in 2012.

As for in-vitro fertilization, the number of ova which are operated in the first period of treatment is about 5 to 20, and ova of women and sperms of men are collected and then they are treated, fertilized, cultivated, stored and transplanted. At that time, ova and sperms of each patient are distinguished exactly and should be treated, fertilized, cultivated, stored and transplanted, and of course it is not forgiven that they are operated by mistake.

In recent years, technique to cryopreserve fertilized eggs ( embryo ) has improved and the pregnancy rate equivalent to fresh embryo is obtained as to frozen embryo and also a merit capable to set up transplant time to uterus flexibly is recognized. Therefore, the ratio of in-vitro fertilization using frozen embryo is increasing greatly.

The state in which an ovum fertilized is called a fertilized egg, and the fertilized egg is divided into 4 cells aged embryo after two days and is further divided, and then the embryo will be transplanted to uterus.

A method to freeze and store the embryo in the state of being ready to transplantation becomes the mainstream on infertility treatments at the present time.

At the time of cryopreserving, sperms are injected into a container for storing sperms such as a specimen tube, a straw and the like, and ova and fertilized eggs are preserved on a container for storing ova such as a cryotop [ Registered Trademark owned by Kitazato Supply Co. Ltd. ] having a sheet portion, a cryoloop having a loop portion and the like.

Then the plural containers for storing sperms and the plural containers for storing ova are maintained on a cane, the plural canes which maintain the plural containers for storing sperms and the like are maintained in a canister, and the plural canisters are stored in a tank for cryopreserving and preserved ( see Patent Literatures 1 and 2 ).

Here, liquid nitrogen is filled up in the tank for cryopreserving and the subjects are cryopreserved under extreme lower temperature of -196 °C at an atmosphere of liquid phase or gas phase.

Conventionally, it was not allowed to cryopreserve unfertilized eggs to execute in-vitro fertilization in the future, but recently, various laws were revised to become allowed to cryopreserve unfertilized eggs for the said purpose. As the result, it is expected that cases in which unmarried women cryopreserve her own ova are increasing.

At the freezing and preserving as mentioned above, so far, information data such as a specimen number, a patient name, an extracted day and time and the like was wrote by one's hand on the end portion of a container for storing sperms, a container for storing ova, a cane and the like, or labels on which the information was mentioned were put on the end portion of them. Then embryo cultivators recognize the wrote point of the containers and instrument with his eyes or the labels put on them with his eyes so as to distinguish which patient's sperms or ova.

Further, labels on which a bar code was printed were put on a container for storing sperms, a container for storing ova, a cane and the like, and then embryo cultivators read the bar code with a bar code reader so as to distinguish which patient's sperms or ova.

### Citation List

### Patent Literature

Patent Literature 1: JP 2015-089396 A
Patent Literature 2: JP 2016-067807 A

### Summary of Invention

### Technical Problem

By the method as mentioned above in which information data such as a specimen number, a patient name, an extracted day and time and the like was written by one's hand on the end portion of a container for storing sperms, a container for storing ova, a cane and the like, or the method as mentioned above in which labels on which the information was mentioned were put on the end portion of them, embryo cultivators recognize the written point of the containers and instrument or the labels put on them with his eyes, and therefore, there is a possibility that the case of operating a specimen which is an object for treatment by mistake may occur.

On the other hand, the plural containers for storing sperms, the plural containers for storing ova and the plural canes are maintained in a canister, and the plural canisters are stored in a tank for cryopreserving which is filled up with liquid nitrogen and preserved under the environment of -196 °C. Therefore, when the containers and instruments are took out from the tank for cryopreserving, frost and the like adhere on them or on the label, unless the frost is stripped off or melted, embryo cultivators can not recognize a specimen number and the like. As the result, it take a long time for embryo cultivators to recognize a specimen number and the like so as to lower the working efficiency and the frost becomes a factor which causes embryo cultivators to operate a wrong specimen by human mistake.

Also in the case that a label on which a bar code is printed is adhered, when frost and the like is adhered, the bar code can not be read. Therefore, it is necessary to stripped off or melt the frost and the work takes a long time. In addition, work for putting a label on which a long and narrow bar code is printed on a correct position of a container and an instrument so as to be able to read is delicate and difficult work because a portion to be put a bar code on is narrow and small in width.

Further, in the case that it takes a long time until embryo cultivators take off the containers for storing sperms, the containers for storing ova and the canes from the tank for cryopreserving and recognize a specimen number and the like on the containers, instrument or the label, sperms and fertilized eggs are placed under the normal temperature during the time, and the internal temperature of them increases and they are easy to change their nature. As the result, this becomes a factor to decrease the fertilization ratio and the implantation ratio.

Furthermore, so far, at the time that embryo cultivators execute the work such as extraction, treatment, fertilization, cultivation, freezing and preserving, melting, implantation and the like of ova and sperms, he enters the data in recording papers or the recording display of a personal computer and record the data. However, patients who were executed the treatment can not know information in the progress of the treatment without the timing of consultation because the state of the treatment is not specified timely to the patients.

The present invention has been made in order to resolve such problems as mentioned above, and it is an object of the present invention to provide an individual management system which is suitable for individual management of reproductive cells such as sperms, ova, fertilized eggs and the like to be cryopreserved, and which prevents confusion of sperms, ova, fertilized eggs and the like which are objects of treatment, improves work efficiency of embryo cultivators greatly, controls decrease of fertilization ratio and implantation ratio, realizes the traceability ( history tracing possibility) to record all working of treatment including working objects, working details, workers and working day and time, and further by which patients can know information in the progress of treatment easily.

As a similar territory field, cryopreservation of sperms, ova, fertilized eggs of animals is also executed widely. In the territory field, as the same management system is adopted, there exist the same problems as in the case of human fertilization.

Therefore, it is also an object to provide an individual management system which is suitable for individual management of reproductive cells such as sperms, ova, fertilized eggs and the like of animals.
US 2016/026911 A1 discloses an identification code reader used in an appropriate place within a facility; an identification code / IC tag reader; a device, an instrument, an implement and a container used to cryopreserve an appropriate subject; and an identification code and an IC tag which are at least attached to or mounted in the device, the instrument, the implement and the container.
JP 2015 222 539 A describes a cell storage system for storing, in a distributed manner in a plurality of cell storage devices installed in different facilities or different rooms, or in the same room, a plurality of containers containing cells of the same type such as egg cells, sperm cells and fertilized egg cells, where there has been a request for storage of such cells by a requester such as a hospital or a research facility, said system comprising: a plurality of manager terminal devices possessed by a manager managing the cells; a cell storage terminal device installed at the periphery of the cell storage devices for storing and managing the cells of the same type; a server device; the plurality of containers are provided with a label on which is recorded a code such as a barcode or a two-dimensional barcode, for example, information such as a container ID is recorded on the recording medium, a code reader able to read the barcode or the two-dimensional barcode including the information such as the container ID, for example, and a recording medium issuing device for printing the code such as a barcode or a two-dimensional barcode on a label.
JP 2007 066011 A discloses a cryopreservation straw with an RFID tag.

### Solution to Problem

In order to achieve the above object, the invention provides an individual management system according to claim 1.

The system comprises a server installed in an appropriate place within a facility, a plurality of personal computers, identification code readers used in an appropriate place within a facility, identification code / IC tag readers, devices, instruments, implements and containers used to cryopreserve specimens, identification codes and IC tags which are attached to or mounted to at least the devices, instruments, implements and containers.

Here, label printers are installed in an appropriate place within a facility, and the devices, the instruments, the implements and the containers installed and used within the facility are connected via intra-facility network.

Further, it is preferable that the intra-facility network is connected to a cloud server via internet and a user of the facility can access the cloud server by a portable information terminal device.

The portable information terminal device is, example, a mobile phones, a smart phone or a tablet computer.

The facility is a facility to execute in-vitro fertilization.

The facility may be a facility to preserve and manage a reproductive cell of an animal.

The device, the instrument, the implement and the container used to cryopreserve an appropriate subject in liquid nitrogen include at least a cryopreservation tank, a canister, a cane and a ovum preservation container.

The cane is a cane on the grasping portion of which an IC tag for cane can be detachably mounted.

The IC tag for cane may have a latch structure and when the IC tag for cane is inserted in the grasping portion of the cane, a latch portion of the IC tag for cane bites in the grasping portion of the cane and the IC tag for cane is not easily dislocated out of the grasping portion of the cane.

The surface of the IC tag for cane may be colored, or an ID may be printed on or a label in which an ID is printed is attached to the surface of the IC tag for the cane.

The IC tag for ovum preservation container in which an inlet is contained in a cover pipe is mounted on an upper end of a rod-like part of the ovum preservation container.

Here, an internal diameter of a middle part of the cover pipe is smaller than a diameter of a circumscribed circle in the rod-like part of the ovum preservation container.

A hole or a groove is formed in the inlet in an axial direction.

The individual identifying information of a fertilized egg and information concerning a fertilized egg are stored in the inlet.

Further, a type code and a color code of the container may be stored in the inlet.

A surface of the IC tag of the ovum preservation container may be colored or the inlet of the IC tag of the ovum preservation container may be colored.

An ID is printed on or a label in which an ID is printed is attached to the surface of the ovum preservation container.

An ID is printed on or a label in which an ID is printed is attached to the surface of the IC tag for the ovum preservation container.

An IC tag for canister is suspended from a hooking portion of the canister.

The device, the instrument, the implement and the container further include a sperm preservation container.

The sperm preservation container is a specimen tube and an IC tag for specimen tube is mounted in a leg portion of a body of the specimen tube.

The sperm preservation container is a sperm preservation straw and an IC tag for sperm preservation container is mounted in a thin pipe of the sperm preservation straw.

An extended antenna can be attached to the identification code / IC tag reader.

The extended antenna comprises a holding member to hold an IC tag, an antenna element fitted into the holding member and a connecting cable capable to be connected to the identification code / IC tag reader.

Here, the antenna element has a shape of cylindrical curved surface.

The extended antenna may comprise an antenna element of wound coil and a connecting cable capable to be connected to the identification code / IC tag reader.

### Advantageous Effects of Invention

According to the individual management system of the present invention, it is possible to determine the order quantity in a long-term view taking into consideration the inventory at the store and readily determine the order quantity even by the inexperienced person who has worked at a store for a little while.

Further, in the case that a sperm preservation container, an ovum preservation container, a cane and the like are taken out from a cryopreservation tank, even if frost and the like are attached to the surface of them, information can be read by an identification code / IC tag reader. Therefore, ID, a specimen number and the like can be recognized without removing frost and the like, and working efficiency of the embryo cultivator can be greatly improved.

It takes a little time to take out a sperm preservation container, an ovum preservation container, a cane and the like from the cryopreservation tank and recognize ID, a specimen number and the like by the embryo cultivator, or else, it is not necessary to take out them from liquid nitrogen.

Therefore, sperms, ova, and the like does not change in quality rarely in the working time, after all, the fertilization ratio and the implantation ratio can be improved greatly.

Further, in the individual management system of the invention, in the case that an embryo cultivator executes work of collection, treatment, fertilization, cultivation, preservation, implantation and the like of ova and sperms, he enters the information in the recording display of a personal computer and stores the information in the personal computer. Therefore, a patient can recognize the information in appropriate time by a portable phone and the like and thereby reliability of treatment can be improved.

Further, in the individual management system of the invention, traceability of all works in the treatment is realized and visualization in process of the treatment is realized. Thereby, safety of the treatment can be improved and guaranteed.

As a result mentioned above, processing ability and working ability of an embryo cultivator become clear, effectivity of cultivation liquid depended on individual variation of fertilized eggs also become clear, and then, by improving the matter, pregnancy rate of infertility treatment can be expected to improve greatly.

The individual management system of the invention can be adopted to the case in which cryopreservation or in-vitro fertilization of sperms, ova, and the like of animals such as a cow, a horse and the like is executed, without human case.

### Brief Description of Drawings

FIG. 1 is a rough configuration view of an individual management system of the present invention.
FIG. 2 is a network diagram of an individual management system of the present invention.
FIG. 3 (A) is a perspective view and (B) is a plan view of a dish.
FIG. 4 is an explanatory view showing a state of cultivating a fertilized egg.
FIG. 5 (A) is a front view and (B) is a sectional view showing the state of cryopreserving sperms, ovum, fertilized eggs and the like in a tank for cryopreserving which is filled up with liquid nitrogen.
FIG. 6 is a front view of a canister.
FIG. 7 is a front view of a cane and an ovum preservation container.
FIG. 8 is a perspective view of one embodiment of an ovum preservation container of the present invention.
FIG. 9 is a perspective view of an ovum preservation container and a protection sleeve.
FIG. 10 (A) is a front view and (B) is a sectional view of a part which an inlet is mounted on in an IC tag for ovum preservation container.
FIG. 11 is a front view showing the state of an ID printed on a rod-like portion of the ovum preservation container.
FIG. 12 is a perspective view showing the state of a label printed an ID attached to a rod-like portion of the ovum preservation container.
FIG. 13 is a front view showing the state of an ID printed on a cover pipe of an IC tag for ovum preservation container.
FIG. 14 is a front view showing the state of a label printed an ID attached to a cover pipe of an IC tag for ovum preservation container.
FIG. 15 is a front view of other embodiment of an ovum preservation container of the present invention.
FIG. 16 (A) is a front view of a state of separating of a formation jig forming a thin-walled pipe comprising an IC tag shown in FIG. 15 and (B) is a longitudinal sectional view of a state of fitting together.
FIG. 17 is an explanatory view showing a method of forming the thin-walled pipe comprising the IC tag shown in FIG. 15.
FIG. 18 is an explanatory view showing a method of forming the thin-walled pipe comprising the IC tag shown in FIG. 15.
FIG. 19 is a perspective view of a cane and an IC tag for cane.
FIG. 20 (A) is a bottom perspective view and (B) is a top perspective view of an IC tag for cane.
FIG. 21 is a perspective view showing the state of an ID printed on a surface of the IC tag for cane.
FIG. 22 is a perspective view showing the state of a label printed an ID attached to a surface of the IC tag for cane.
FIG. 23 is a sectional view of a specimen container.
FIG. 24 is a perspective view showing the state holding multiple specimen containers in the cane.
FIG. 25 is a front view of a conventional straw.
FIG. 26 is a front view of a straw mounted an IC tag for straw on.
FIG. 27 is a perspective view of an identification code / IC tag reader.
FIG. 28 (A) is a usage state view and (B) is an enlarged view of main part of one embodiment of an extended antenna attached to the identification code / IC tag reader shown in FIG. 27.
FIG. 29 is a usage state view of other embodiment of the extended antenna attached to the identification code / IC tag reader shown in FIG. 27.
FIG. 30 is a rough configuration view of the extended antenna attached to the identification code / IC tag reader shown in FIG. 27.
FIG. 31 (A) is a front view and (B) is a perspective view of other embodiment of the extended antenna attached to the identification code / IC tag reader shown in FIG. 27.
FIG. 32 (A) is a front view and (B) is a perspective view of other embodiment of the extended antenna attached to the identification code / IC tag reader shown in FIG. 27.

### Description of Embodiments

Preferred embodiments of an individual management system of the present invention in the case applied to in-vitro fertilization will be described in detail below with reference to the drawings.

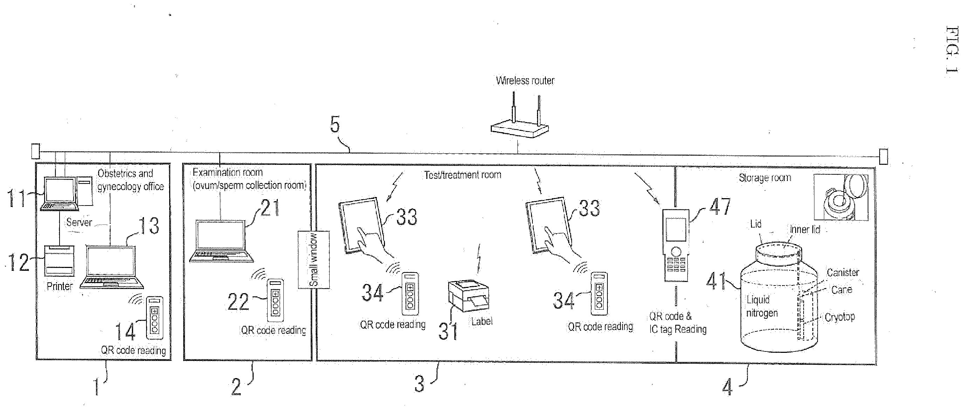

As shown in FIG. 1, an individual management system of the present invention comprises a server 11, a printer 12 and a personal computer 13 installed in the office 1, an identification code reader 14 which is used in the office 1, a personal computer 21 installed in the examination room 2, an identification code reader 22, a label printer 31 installed in the test / treatment room 3, a tablet computer 33 and identification reader 34 which are used in the test / treatment room 3, a tank for cryopreservation 41 installed in the storage room 4, devices, instruments, implements and containers such as canisters 42, canes 43, preservation containers 44 for ova which are stored in the tank for cryopreservation 41, and identification code / IC tag readers which are used in the storage room 4.

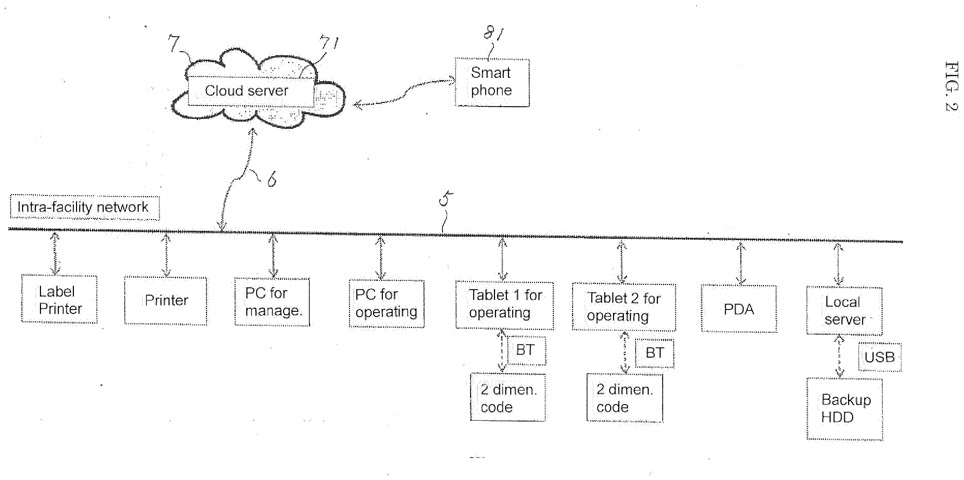

And in the individual management system of the invention, as shown in FIG. 2, the devices, the instruments, the implements and the containers installed or used in these facilities are connected via intra-facility network 5.

Further, they are connected to a cloud server 71 installed in a cloud provider 7 via internet 6 and the system can be applied.

In this case, a patient under treatment for in-vitro fertilization can read designated information from the cloud server 71 by a mobile phone, a smart phone, a tablet computer and the like which are used himself under the designated condition.

Various kind of devices, instruments, implements and containers mounted IC tags are used in the individual management system of the invention. In the case that the subject managed by the individual system of the invention is applied not only to reproductive cells of humans but also to reproductive cells of animals, more various kind of devices, instruments, implements and containers mounted IC tags are used.

Therefore, information including properties such as a kind of devices, instruments, implements and containers mounted IC tags, a color painted on the surface of IC tags and the like is stored in IC tags. By using such kind of property information in the individual management system at the time that various works are executed, the efficiency and the ease of use of the system can be improved greatly.

Examples of the property information which is stored in IC tags are shown in Table 1 and 2.

**[Table 1]**

| humans /animals | kinds of tags | kinds of codes | color codes | information of patients/animals |
|---|---|---|---|---|
| common | common | 0 | 0 | |
| humans | tank | 1 | 0 | |
| | canister | 2 | 0 | |
| | cane | 3 | 1,2,3,4,5,6 | |
| | cryotop | 4 | 0,1,2,3,4,5,6 | |
| | specimen tube | 5 | 1,2,3,4,5,6 | |
| animals | straw | 6 | 0,1,2,3,4,5,6 | |
| | sub-canister | 7 | 0 | |
| | cell | 8 | 1,2,3,4,5,6 | |

**[Table 2]**

| color | non (0) | blue (1) | green (2) | yellow (3) | white (4) | red (5) | purple (6) | green/red (7) |
|---|---|---|---|---|---|---|---|---|
| color codes | 0 | 1 | 2 | 3 | 4 | 5 | 6 | 7 |

Next, a concrete construction and function of the above-mentioned devices, instruments, implements and containers will be described according to the treatment process of in-vitro fertilization.

At the time that the execution of in-vitro fertilization is determined, an office worker enters a patient number ( ID ), patient name and the like in a personal computer 13 to show patient information on a display, executes the prescribed operation to make an instruction for collecting eggs, and prints the instruction for collecting eggs by a printer 12.

In the case that the individual management system is connected to an electronic medical record system, by entering a patient number ( ID ) into the personal computer 13, necessary patient information can be received from the electronic medical record system

By the way, a patient number ( ID ), a patient name, a instruction number and the like are printed in the instruction for collecting eggs and 2-dimensional code tied to the information is also printed in it.

At the time that the schedule for collecting eggs which is a treatment of in-vitro fertilization is determined, a person in charge prepares containers which are used in the treatment in advance, and at the same time, prints a patient number ( ID ) and a patient name on labels, and also prints 2-dimensional code having the patient number ( ID ), the patient name, the instruction number and the container number ( ID ) on the labels by a label printer 31.

The labels are labels which are attached to containers. The person in charge prints the same number of labels as the number of the containers in advance and attaches the labels to the containers.

Further, the person in charge also prints a label which is attached to a wrist band wounded around a patient wrist, and attaches the label to a designated part of the wrist band. Then, on the appointed day when the collecting eggs is executed, the person in charge confirms the patient to be the patient herself and mounts the wrist band to the patient.

Next, in an examination room 2, a nurse and the like reads the instruction, the label attached to the container for collecting eggs in advance and 2-demensional code of the wrist band of the patient by an identification code reader 22, and confirms to be correct.

In the examination room 2, a doctor and the like collects follicles from the patient and inserts the follicles into the container for collecting eggs confirmed as mentioned above. After that, the nurse and the like delivers the container for collecting eggs in which the follicles are inserted and the instruction to the embryo cultivator immediately.

Next, in the test / treatment room 3, the embryo cultivator takes out the follicles from the container for collecting eggs received, and checks the number of ova.

Then, the embryo cultivator reads the instruction and 2-demensional code printed on the label which is attached to the container for collecting eggs by an identification code reader 34 to confirm the patient and the container for collecting eggs, and enters and records the number of ova by a tablet computer 33.

In the case that the ova checked the number are moved to an another container, the embryo cultivator reads the 2-demensional code of the former container and the 2-demensional code of the container to which the ova are moved, and enters the number of the ova moved in the tablet computer 33 and records the movement work.

The information as mentioned above is transmitted to a server 11 installed in the office 1 via intra-facility network 5 and is stored in the server 11.

Whereby, the doctor and the like can confirm the number and the state of ova by reading the information stored in the server 11 installed in the office 1 with the personal computer 21 installed in the examination room 2 via intra-facility network.

Next, the embryo cultivator executes various treatments to the ova, inserts the ova after the treatments to a preservation container, and preserves the ova within an incubator.

As for these works, recording and management of the preservation place of ova and the like are executed by reading the 2-dimentional code of a preservation container.

At the time that the execution of fertilization is determined, generally, conventional in-vitro fertilization ( IVF ) in which sperms are sprinkled over ovum to fertilize the ovum is executed.

Further, intra-cytoplasmic sperm injection ( ICSI ) in which sperms are injected to egg cytoplasm of ovum by a micro pipette to fertilize the ovum is executed.

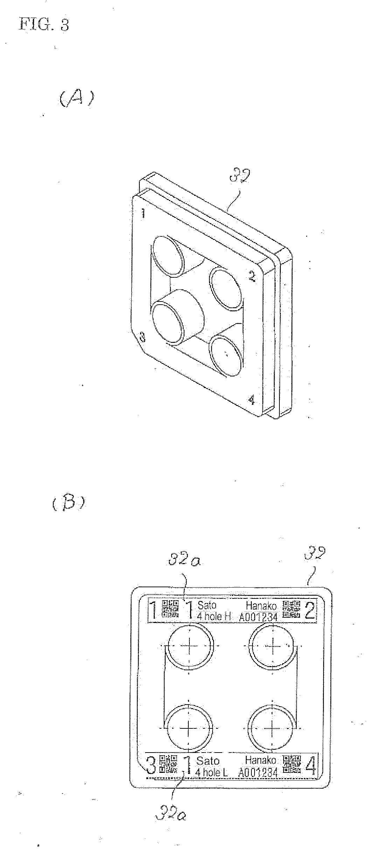

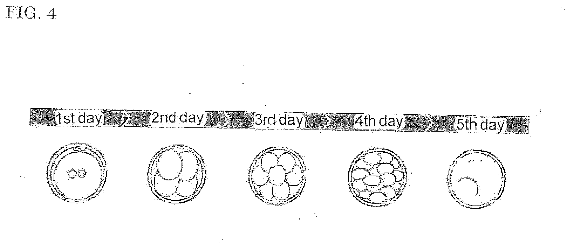

After that, within the incubator, as shown in FIG. 3, a fertilized egg is cultivated with culture fluid in a dish 32 and after 2 or 3 days has passed, as shown in FIG. 4, the fertilized egg becomes to be a divided embryo divided into 4 or 8 cells. The cultivation of the divided embryo is continued further and after 5 or 6 days has passed, the divided embryo grows an blastodermic vesicle in which the existence of inner cell mass is recognized.

After fertilization, the individual management of a fertilized egg is executed by tying ID of the ovum to 2-demensional code of the container individually.

In the process, the embryo cultivator reads 2-dimensional code printed in the label 32a attached to the dish 32 by an identification code reader 34 to identify the fertilized egg to be the subject, and records the cultivation state of the fertilized egg.

Whereby, information of the cultivation state of the fertilized egg is transmitted to the server 11 via intra-facility network and stored in the server 11.

Accordingly, the information can be read from the personal computer 21 installed in the examination room 2 and the personal computer 13 installed in the office 1 by accessing the server 11.

Next, the doctor and the like executes an initial embryo transfer in which the split embryo divided to 4 or 8 cells after 2 or 3 days cultivation is returned to the mother's body in the examination room 2. Or else, the doctor and the like may executes blastocyst implantation in which the blastocyst that is recognized existence of inner cell mass is returned to the mother's body.

In this process, the doctor and the like reads 2-demensional code printed in the label 32a attached to the dish 32 by the identification code reader 22 to identify the fertilized ovum to be the subject, and enters implantation information in the personal computer 21.

Whereby, implantation information of the fertilized egg is transmitted from the personal computer 21 installed in the examination room 2 to the server 11 via intra-facility network 5 and restored in the server 11.

In the case that the fertilized egg is cryopreserved, the fertilized egg which is in the state capable to execute implantation is cryopreserved.

In order to lighten the burden of the mother body owing to collecting eggs, enough number of ova capable to execute plural times of in-vitro fertilization are collected at one time. And then, by cryopreserving enough number of fertilized eggs capable to execute embryo transfer at plural time, the fertilized eggs are not transferred at the time when uterus environment is not suitable to transfer in the cycle of collecting eggs because of the influence of fertility drug and the like, and the fertilized egg is transferred at the time when the state of mother body is returned to the normal state after the next cycle. Whereby, implantation ration and pregnancy ratio are improved.

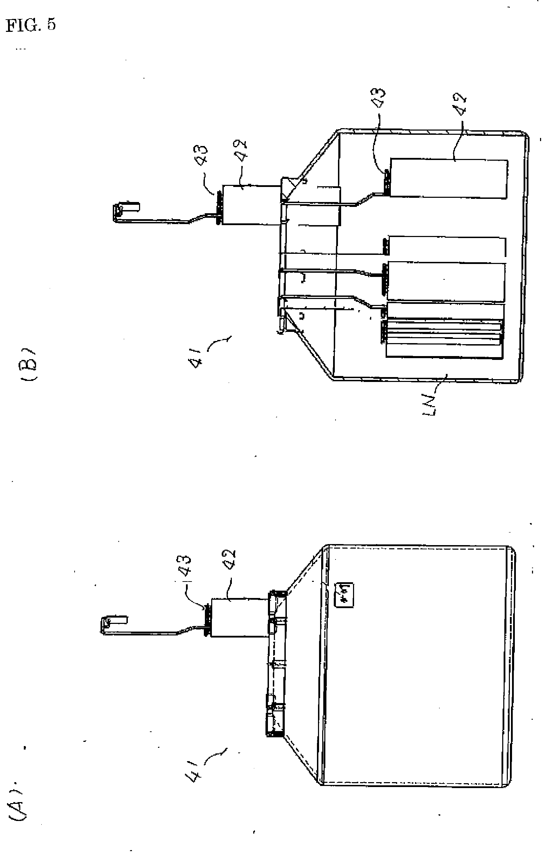

As shown in FIG. 5 to 8, devices, instruments, implements and containers used in cryopreservation are a tank for cryopreservation 41, canisters 42, canes 43, specimen tubes 44, ova preservation containers 45, protection sleeves 46 and the like.

As shown in FIG. 5, the tank for cryopreservation 41 comprises a body, an external lid and an internal lid, and liquid nitrogen LN of -196 °C is filled up within the tank for cryopreservation 41

About 6 to 12 canisters 42 are suspended and supported radially within the tank for cryopreservation 41, about 10 to 20 canes are supported in a canister 42.

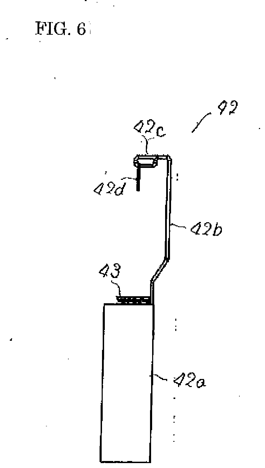

As shown in FIG. 6, the canister 42 comprises a cylindrical portion 42a, a rod portion 42b, a suspended portion 42c and IC tag for canister 42d.

The plural canes 43 are inserted into the cylindrical portion 42a, by suspending the suspending portion 42c at the internal lid of the tank for cryopreservation 41, the canisters 42 are supported within the tank for cryopreservation 41.

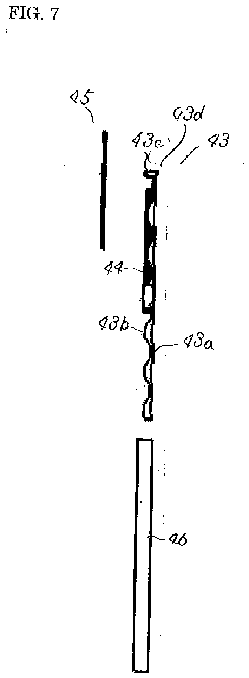

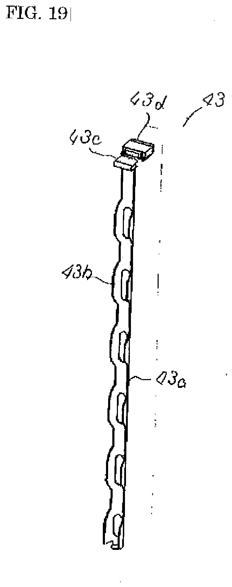

As shown in FIG. 7 and 19, the cane 43 comprises a rod portion 43a, a support portion 43b, a grip portion 43c and IC tag for cane 43d.

The specimen tubes 44 capable to store plural ovum preservation containers 45 can be fitted and suspended in the support portion 43b, and the ovum preservation containers 45 can be supported in liquid nitrogen LN.

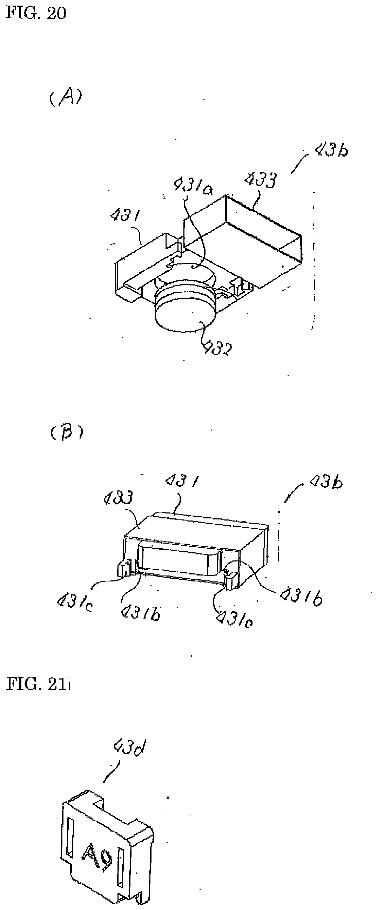

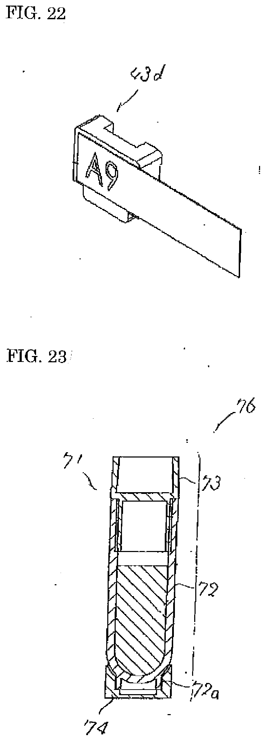

As shown in FIG. 20 (A), the IC tag for cane 43d is formed to insertion structure, as shown in FIG. 22 (A), the IC tag for cane 43d comprises a tag case 431, a flex IC tag 432 which is fitted in a fitting hole 431a of the tag case 431 and label 433 wounded around the tag case 431.

As shown in FIG. 20 (B), an inserting groove portion 431b is formed in the tag case 431, nail portions 431c, 431c are formed in the both side of the inserting groove portion.

And the inserting groove portion 431b and the nail portions 431c, 431c form so-called a latch structure.

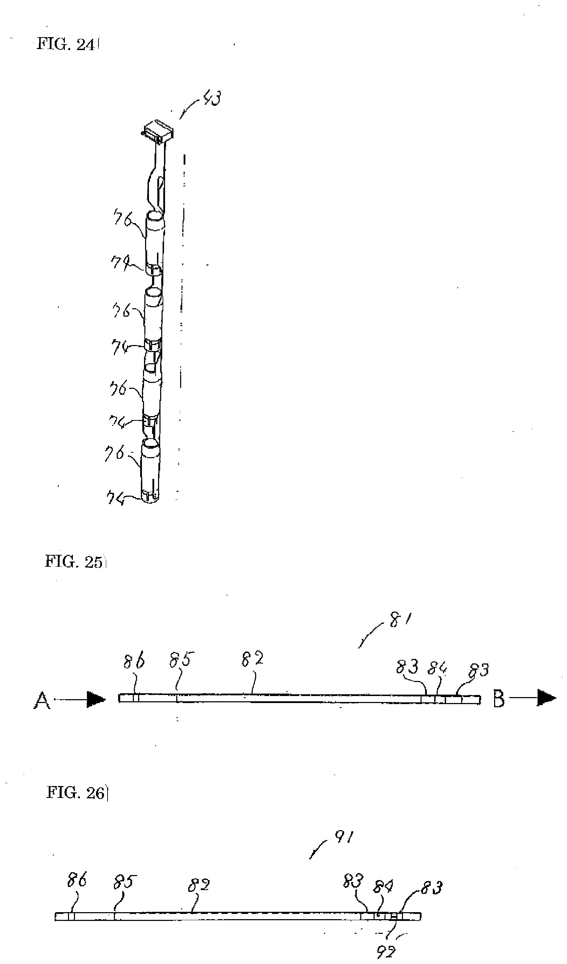

As shown in FIG. 19, by inserting the grip portion 43c of the cane 43 into the insertion groove portion 431b of the IC tag for cane 43d, as shown in FIG. 24, the IC tag for cane 43d is mounted on the grip portion 43c.

Here, at the time that the grip portion 43c of the cane 43 is inserted to the insertion groove portion 431b of the IC tag for cane 43d the once, nail portions 431c, 431c are engaged. Therefore, the IC tag for cane 43d is not easily omitted from the grip portion 43c.

An external mounting material of IC tag 43d is painted with determined color, thereby the cane 43 can be distinguished. Classification way by color may be a classification with one color or may be a classification with multi-color. Further, the cane 43 may be distinguished by forming a various patterns of color.

Considering the case in which there are only limited number of color and the same color is used repeatedly, as shown in FIG. 21, ID may be directly printed on the surface of the external mounting material of IC tag for cane 43d, or as shown in FIG. 22, a label in which ID is printed may be attached to the surface of the external mounting material to distinguish the cane 43.

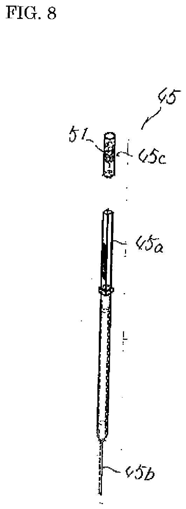

As shown in FIG. 8, the ovum preservation container 45 which preserves ova or fertilized eggs comprises a rod portion 45a of square pillar and a support plate 45b made of Plastics.

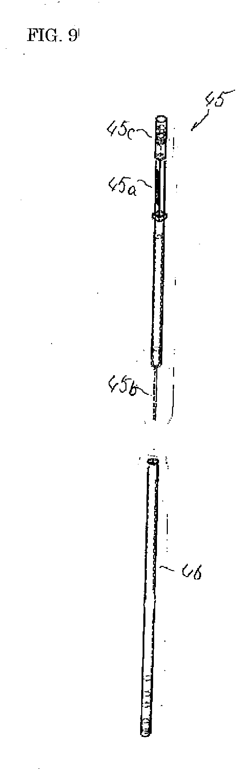

The fertilized egg is mounted on the edge portion of the support plate 45b with a little preservation liquid, and is frozen suddenly in liquid nitrogen, as shown in FIG. 9, and then the ovum preservation container 45 is stored in a protection sleeve 46.

This preservation method is called vitrification and is a main stream of cryopreservation method of fertilized egg because it is possible to cryopreserve a cell without injure by vitrifying ( a phenomenon of not forming an ice crystal and becoming a vitreous solid of non-crystallization ) the cell together with preservation liquid.

As shown in FIG. 8, an IC tag for ovum preservation container 45c is mounted on the upper edge portion of the rod-like portion 45a of the ovum preservation container 45c.

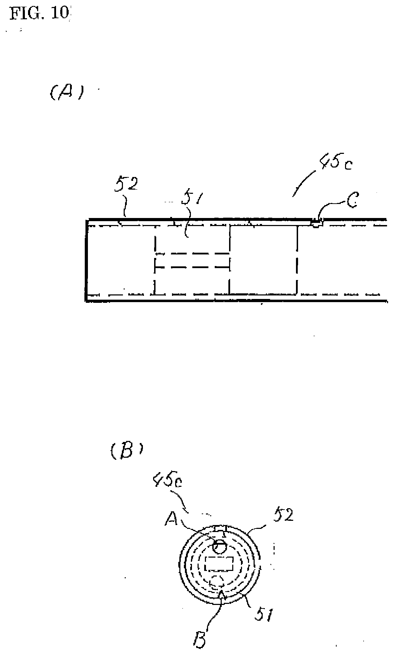

As shown in FIG. 10, the IC tag for ovum preservation container 45c is constituted by containing an inlet 51 of cylindrical shape having a micro diameter into a cover pipe 52 made of plastic.

And then, the IC tag for ovum preservation container 45c is fixed and mounted on the upper end portion of the rod-like portion 45a of the ovum preservation container 45c by filling up synthetic resin or filler into the cover pipe 52.

The ovum preservation container 45 is inserted in the cryopreservation tank 41 and is preserved under the environment of liquid nitrogen LN at -196°C. After that, when the ovum preservation container 45c is took out from the cryopreservation tank 41 and is maintain under the environment of normal temperature at 20 °C, liquid nitrogen LN which invades a gap between the upper portion of rod-like portion of the ovum preservation container 45 and the IC tag for ovum preservation container 45c vaporizes and the volume expands suddenly to be nitrogen gas N₂.

Whereby, the IC tag for ovum preservation container 45c is loaded with an excessive pressure and it is feared that the IC tag for ovum preservation container 45c jumps out from the rod-like portion.

Therefore, in the present invention, as shown in FIG. 10, a hole or a groove is formed in the inlet 51 of the IC tag for the ovum preservation container 45c in the axial direction.

Whereby, even if liquid nitrogen LN vaporizes and expands suddenly in volume to be nitrogen gas N2, it is not feared that the IC tag for ovum preservation container 45c jumps out from the rod-like portion, because nitrogen gas N2 is released through the hole A or the groove B.

As shown in FIG. 10, a hole C for venting gas may be formed in the peripheral surface of the cover pipe 52 of the IC tag for ovum preservation container 45c, or a narrow and long slit may be formed in the axial direction in the peripheral surface of the cover pipe 52.

By such a configuration, the action and the effect as mentioned above are also taken.

The rod-like portion 45a is painted with a predetermined color and thereby the ovum preservation container 45 can be also distinguished.

Further, in case that the cover pipe 52 of the IC tag for the ovum preservation container 45c or the inlet 51 is painted with predetermined color, many subjects can be distinguished by eyes using the combination of the color as mentioned above and the color of the rod-like portion 45a.

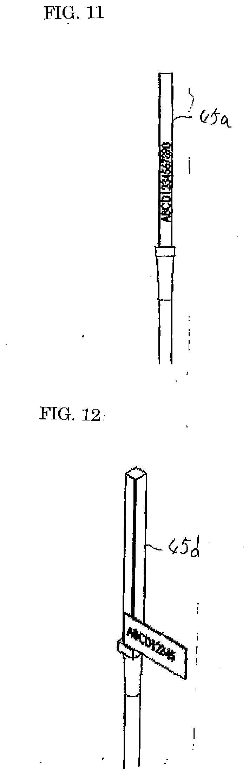

As shown in FIG. 11, ID may be directly printed on the surface of the rod-like portion 45a of the ovum preservation container 45, or else, as shown in FIG. 12, a label printed ID is attached to the surface of the rod-like portion 45a to distinguish the subjects.

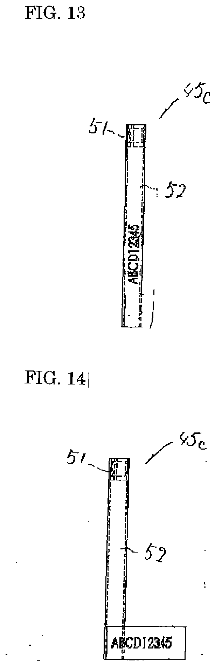

Further, as shown in FIG. 13, ID may be directly printed on the surface of the cover pipe 52 of the IC tag for the ovum preservation container 45c, or else, as shown in FIG. 14, a label printed ID is attached to the surface of the cover pipe 52 to distinguish the subjects.

Whereby, in case that the IC tag 45c is broken down and ID cannot be read, or in case that IC tag 45c is dislocated, information of the subject preserved in the preservation container can be restored by reading ID printed as mentioned above by eyes.

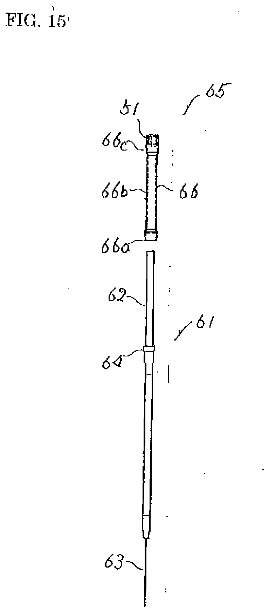

As shown in FIG. 15, another embodiment of IC tag for the ovum preservation container 65 comprises a thin pipe 66 made of plastic, an inlet 51 equipped in the thin pipe 66 and fixing members 52, 52 to fix the inlet 51 from the both side.

And then, as shown in FIG. 15, an internal diameters of an open portion 66a and a close portion 66c are equal to a diameter of a circumscribed circle of the rod-like portion 62 of the ovum preservation container 61 and an internal diameter of the middle portion 66b is a little smaller than a diameter

As the IC tag for the ovum preservation container 65 is such a configuration as mentioned above, it is easy to insert the IC tag 65 into the rod-like portion 62 of the ovum preservation container 61, further, by pushing the IC tag 65 into the rod-like portion 62, the middle portion 66b compresses the rod-like portion 62 and the IC tag 65 can be strongly fixed in the rod-like portion 62

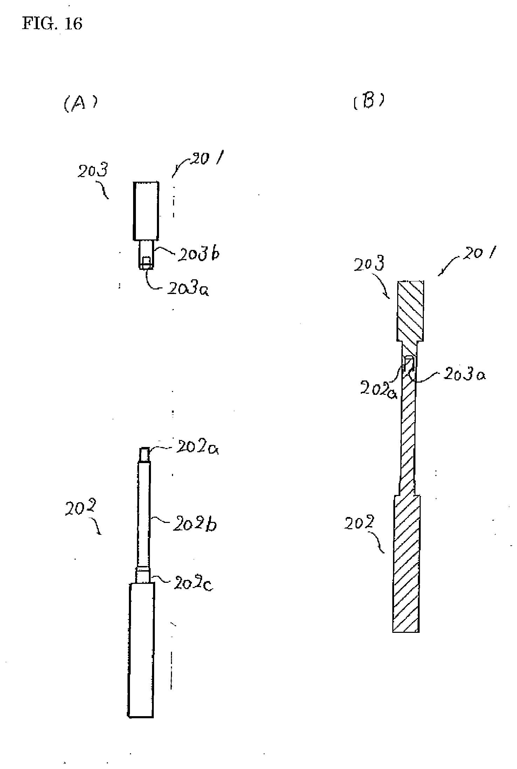

The thin pipe 66 can be formed using a formation jig 201 as shown in FIG. 16. As a material of the thin pipe 66, a material having heat contractility is selected.

As shown in FIG. 16, the formation jig 201 comprises a jig body 202 and a jig head 203, and a tip projection portion 202a of the jig body 202 is inserted into a fitting hole portion 203a of the jig head 203 to be fitted together.

And an external diameter of a middle rod portion 202b of the jig body 202 is a little smaller than an external diameter of the rod-like portion 62 of the ovum preservation container 61, an external diameter of a basic rod portion is almost equal to an external diameter of the rod-like portion 62 of the ovum preservation container 61.

Further, an external diameter of a tip rod portion 203b of the jig head 203 is almost equal to an external diameter of the rod-like portion 62 of the ovum preservation container 61.

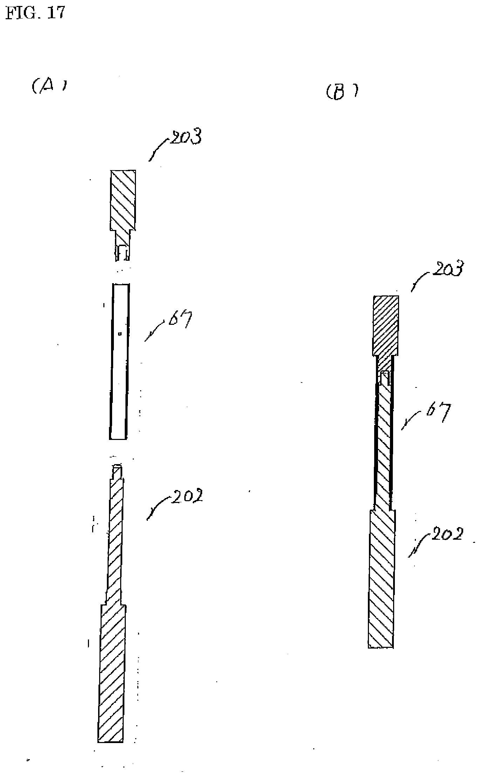

First, as shown in FIG. 17 (A), a raw pipe 67 an external diameter of which is almost equal to an external diameter of the rod-like portion 62 of the ovum preservation container 61 is inserted to the base rod portion 202c of the jig body 202, and then, as shown in FIG. 17 (B), the end rod portion 203b of the jig head 203 is inserted from an end opening of the raw pipe 67 and the fitting hole 203a is fitted on the tip project portion 202a.

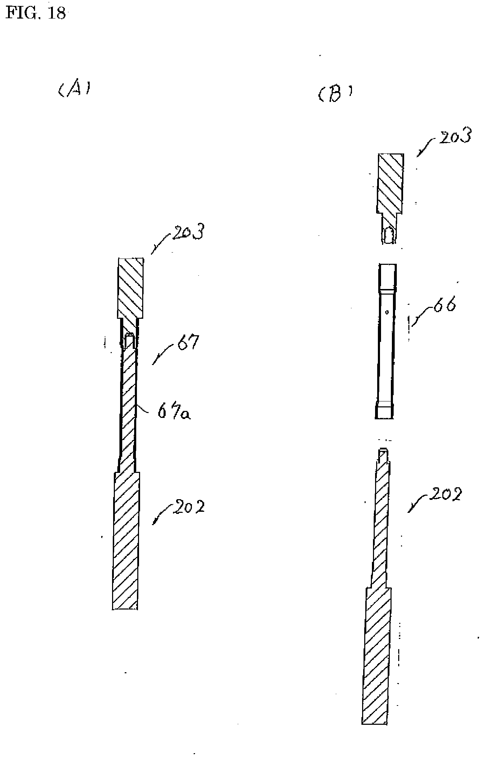

Next, the raw pipe 67 is added to heat of predetermined temperature, as shown in FIG. 18 (A), a middle portion 67a of the raw pipe 67 is contracted and is formed as a shape of the formation jig 201. And then, by separating the jig body 202 from the jig head 203, as shown in FIG. 18 (B), the thin pipe 66 a middle portion of which has a small diameter can be formed.

Conventionally, as a sperm preservation container used to cryopreserve sperms, a specimen tube, a sperm preservation straw and the like are known.

A conventional specimen tube 71 comprises a tube body 72 and a cap 73 screwed on the tube body 72.

As shown in FIG. 23, a specimen tube 76 of the invention 1 is configured by mounting an IC tag for specimen tube 74 in the leg portion 72a of the tube body 72.

Here, an IC tag 74 may be mounted on the cap 73.

As shown in FIG. 24, plural specimen tubes 76, 76, ··· mounted IC tags for specimen tube are fitted on the cane 43 and preserved.

A conventional sperm preservation straw 81, as shown in FIG. 25, comprises a thin pipe 82 made of plastic, a cotton stopper 83, 83 which are loaded in an end portion of the thin pipe 82, and an absorption member 84 which is loaded between the cotton stoppers 83, 83.

Sperms are injected into the thin pipe 82 from the end A while air is absorbed from the end B of the thin pipe 82, and the injection of sperms is stopped when the surface of liquid reaches the line 85.

After that, the other end of the thin pipe 82 is adhered by thermos-compression bonding or ultrasonic wave bonding to form a seal portion 86 and the thin pipe 82 is sealed.

As shown in FIG. 26, a sperm preservation straw 91 of the invention is configured to include IC tag 92 within the cotton stopper 83 in the outside which is a component of the sperm preservation straw 91.

By including the IC tag 92 within the cotton stopper 83, the IC tag 92 is sustained without preventing the ventilation.

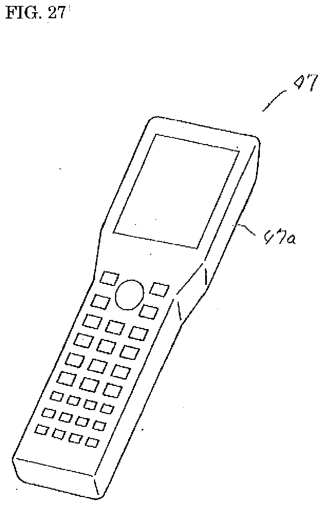

In the storage room 4, an embryo cultivator in working time carries an identification code / IC tag reader 47 as shown in FIG. 27.

When the cryopreservation of fertilized eggs is started, the cryopreservation state is recognized, the melting work is executed, the cryopreservation is finished ( abolition ) and the like, the embryo cultivator puts the antenna 47a of the identification code / IC tag reader 47 close to the IC tag for cane 43b or the IC tag for ovum preservation container 45c which is mounted on the cane 43 or the ovum preservation container 45 and reads ID, a patient information and preservation information which are stored in the IC tag for cane 43b or the IC tag for ovum preservation container 45c.

A connecting ID for reading information which is stored in the data base is stored in the IC tag for cane 43b or the IC tag for ovum preservation container 45c. Further, the patient information, the information of ovum and the preservation information may be stored in the IC tag for cane 43b or the IC tag for ovum preservation container 45c.

Labels printed 2-dimentional code are attached to the other containers used in the work, and the movement of preservation place of the ovum ( ovum ID ) is managed by reading the 2-dimentional code of each container when the ovum contained in the container is moved.

Here, it is easy to read the IC tag for cane 43d mounted on the cane 43 by putting the identification code / IC tag reader 47 close to the IC tag for cane 43d because the IC tag for cane 43d has a large external diameter relatively. However, it is feared not to read the IC tag for ovum preservation container 45c mounted on the ovum preservation container 45 by putting the identification code / IC tag reader 47 close to the IC tag for ovum preservation container 45c because the IC tag for ovum preservation container 45c has a minute external diameter.

Further, the antenna 47a of the identification code / IC tag reader 47 is arranged in the front side or in the back side of the identification code / IC tag reader 47 and it is necessary to read in the state corresponding the IC tag for cane 43d with the location of the antenna 47a, and therefore, the reading work takes a long time.

Therefore, in the invention, an extended antenna 48 can be attached to the identification code / IC tag reader 47.

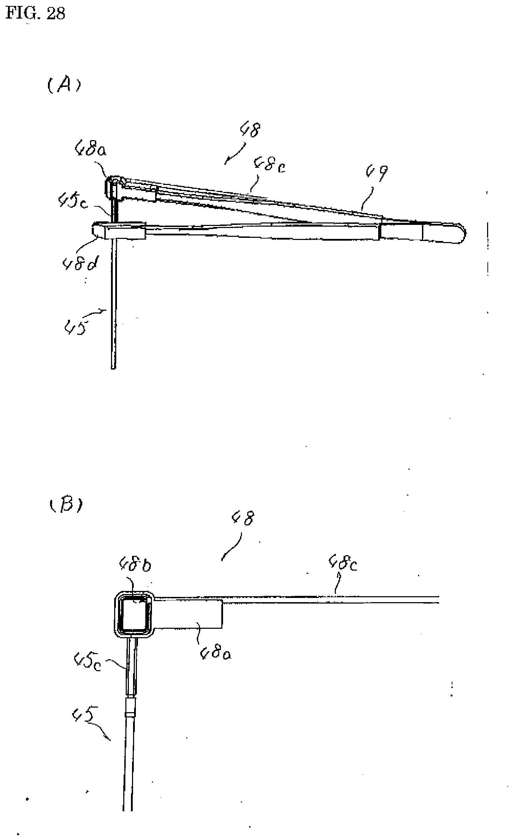

As shown in FIG. 28, the extended antenna 48 comprises a holding member 48a, an antenna element 48b, a connecting cable 48c and a nipping member 48d.

As for the holding member 48a, a fitting groove is formed in the tip end portion of the holding member 48a to fit in and sustain the IC tag for ovum preservation container 45c of the ovum preservation container 45 and a fitting hole is formed in the rear end portion of the holding member 48a to fit in and insert a tip portion of a sustaining tool such as a pair of tweezers 49 and the like.

The antenna element 48b is fitted in the inner side of the tip end portion of the sustaining member 48a and the connection cable is extended out from the one end portion of the antenna element 48b.

As for the nipping member 48d, a fitting hole is formed in the rear end portion of the nipping member 48d to fit in and insert a tip portion of a sustaining tool such as a pair of tweezers 49 and the like.

As shown in FIG. 28, the both tip portions of tweezers 49 are fitted in the fitting hole of the holding member 48a and the fitting hole of the nipping member 48d and the IC tag for ovum preservation container 45c of the ovum preservation container 45 is fitted in the fitting groove of the holding member 48a.

And then, by closing the tip portions of the pair of tweezers 49 and nipping the IC tag for ovum preservation container 45c between the holding member 48a and the nipping member 48d, the antenna element 48b can be received electromagnetic energy from IC tag 45c.

The electromagnetic energy is transmitted to the antenna 47a of the identification code / IC tag reader 47 via the connecting cable 48c after passing through a resonant / coherent circuit 48e. Therefore, information data can be read by the identification code / IC tag reader 47.

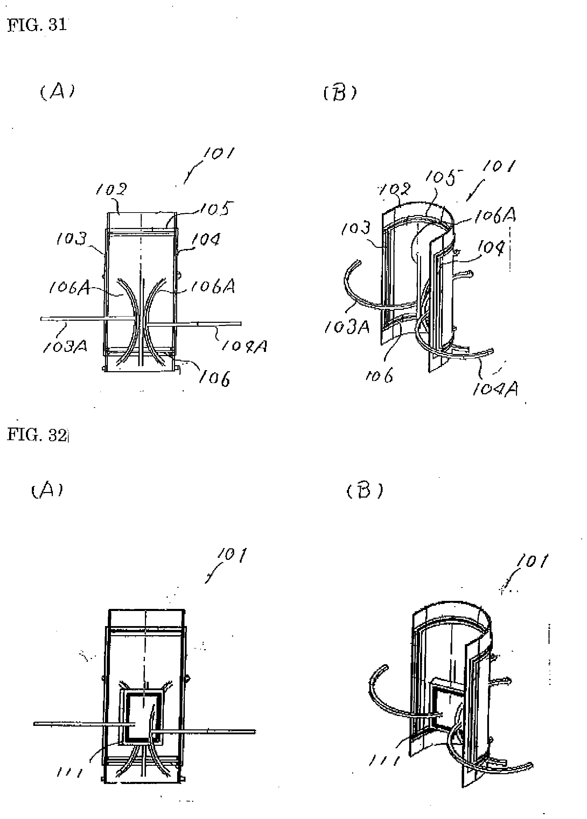

An antenna element 101 which has a thin and curved surface of cylindrical as shown in FIG. 31 may be adopted instead of the antenna element 48b which has a plane surface.

The antenna element 101 is configured by adhering a wire of coil on the thin and curved surface plate of cylindrical 102, and longitudinal elements 103, 104 and lateral elements 105, 106 are configured.

As shown in FIG. 31, since magnetic flux 103A, 104A extend from the longitudinal elements 103, 104 and magnetic flux 106A extends from the lateral element 106, an antenna of the inlet which is positioned inside the antenna element 101 is in the state of being crossed by various directions of magnetic flux.

Accordingly, even if the antenna of the inlet of the IC tag 45c is turned in any direction, reading efficiency is not reduced.

As shown in FIG. 32, when a board stacked inlet 111 having a flat surface is positioned inside the antenna element 101 which has a thin and curved surface of cylindrical, an antenna of the inlet 111 is crossed by various direction of magnetic flux.

Accordingly, as for the board stacked inlet 111, enough reading efficiency can be secured.

In order to read the IC tag for cane 43d, the extended antenna 51 as shown in FIG. 28 may be attached to the identification code / IC tag reader 47.

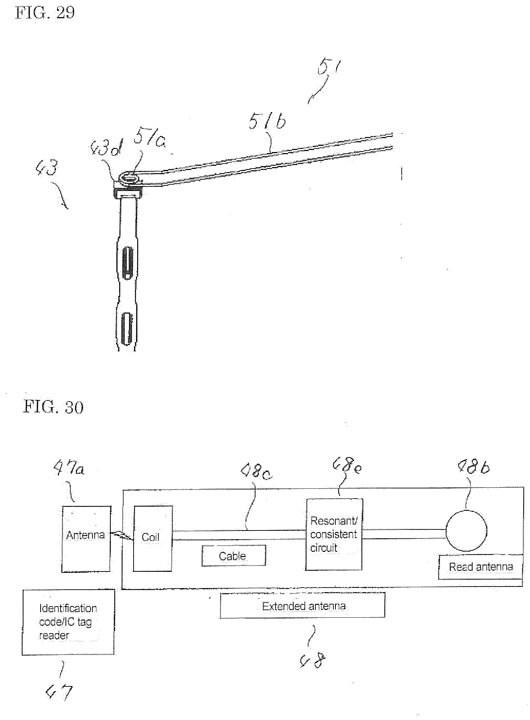

As shown in FIG. 29, the extended antenna comprises an antenna element 51a and a connecting cable 51b.

Here, the connecting cable 51b is electromagnetically connected to the antenna 47a of the identification code / IC tag reader 47 after passing through a resonant / coherent circuit 48e as shown in FIG. 30.

Owing to the extended antenna 51, a tip end portion of the extended antenna 51 can be inserted within liquid nitrogen. Therefore, the IC tag for cane 43d can be read in the state that the cane 43 is sustained in the canister 42 which is suspended within the preservation tank 41 without taking out the IC tag for cane 43d from liquid nitrogen.

As mentioned above, information stored in the IC tag for ovum preservation container 45c and stored in the IC tag for cane 43d and read by the extended antenna 48, 51 is transmitted to the server 11 installed in the office 1 via intra-facility network 5 and is stored in the server 11.

A person in charge, a doctor, an embryo cultivator and the like can recognize patient information, ova information, preservation information in appropriate time by using a personal computer 13 installed in the office and a personal computer 21 installed in the examination room and receiving information from the server 11.

On the other hand, since the server 11 is connected to intra-facility network 5 and is also connected to the cloud server 71 via Internet 6, a patient can require information on himself by using his own portable information terminal device 81 and accessing the cloud server 71.

Here, because patient information, preservation information and the like are especially important personal information, the management of the information must be executed strictly so that a patient cannot require patient information, preservation information and the like without his own information.

Therefore, a patient requests a manager of the individual managing system for the authority to access the cloud server 71 and the manager must examine the request strictly and give the patient the authority to access the cloud server 71. Further, the restriction on a type of information to which a patient can access is also necessary. And also, the system should be configured so that the patient cannot access the cloud server 71 if an identification number and a pass word of his own are not entered.

It is preferable to adopt SSL / TLS protocol such as HTTPS and the like in the data communication and execute the processing of certification to the cloud server 71, encryption of communication content, detection of alteration and the like. As for details of the communication text, an ordinary processing of encryption is executed and any means should be taken so as not to leak information to other person.

In the individual management system of the present invention, IC tags stored ID, specimen number, patient name, collecting day and time and the like are attached to sperm preservation container, ovum preservation container, cane and like, and 2-dimensional code is attached to other containers, and thereby, an embryo cultivator can read the information by an identification code / IC tag reader. Therefore, the confusion of specimens to be the subject of treatment does not occur by mistaking a specimen number and the like.

Further, in the case that a sperm preservation container, an ovum preservation container, a cane and the like are taken out from a cryopreservation tank, even if frost and the like are attached to the surface of them, information can be read by an identification code / IC tag reader. Therefore, ID, a specimen number and the like can be recognized without removing frost and the like, and working efficiency of the embryo cultivator can be greatly improved.

Further, in the case that management in which labels printed a barcode are attached to the containers is executed, the work of attaching the labels to a narrow attaching place carefully is not necessary.

It takes a little time to take out a sperm preservation container, an ovum preservation container, a cane and the like from the cryopreservation tank and recognize ID, a specimen number and the like by the embryo cultivator, or else, it is not necessary to take out them from liquid nitrogen.

Therefore, sperms, ova, and the like does not change in quality rarely in the working time, after all, the fertilization ratio and the implantation ratio can be improved greatly.

Further, in the individual management system of the invention, in the case that an embryo cultivator executes work of collection, treatment, fertilization, cultivation, preservation, implantation and the like of ova and sperms, he enters the information in the recording display of a personal computer and stores the information in the personal computer. Therefore, a patient can recognize the information in appropriate time by a portable phone and the like and thereby reliability of treatment can be improved.

As a result mentioned above, processing ability and working ability of an embryo cultivator become clear, effectivity of cultivation liquid depended on individual variation of fertilized eggs also become clear, and then, by improving the matter, pregnancy rate of infertility treatment can be expected to improve greatly.

The constitution of the individual management system of the invention as described above is a suitable embodiment, and as far as the subject matter of the invention is not departed, various constitutions, devices, instruments can be adopted.

Further, the individual management system of the invention is described in the case adopted to the facility in which an in-vitro fertilization is executed, the individual management system of the invention can be adopted to the case in which cryopreservation or in-vitro fertilization of sperms, ova, and the like of animals such as a cow, a horse and the like is executed, without human case.

### Reference Signs List

1 Office
11 Server
13 Personal computer
14 Identification code reader
2 Examination room ( Treatment room)
21 Personal computer
22 Identification code reader
3 Test / treatment room
31 Label printer
33 Tablet computer
34 Identification code reader
4 Storage room
41 Tank for cryopreservation
43 Cane
43c Grip portion
43d IC tag
45 Ovum preservation container
45a Rod-like portion
45c IC tag
47 Identification code / IC tag reader
48 Extended antenna
48a Holding member
48b Antenna element
48c Connecting cable
48d Nipping member
51 Extended antenna
51a Antenna element
51b Connecting cable
5 Intra-facility network
6 Internet
71 Cloud server
81 Portable information terminal device

## Claims

1. An individual management system for reproductive cells in a facility for in-vitro fertilization which includes an office (1) where an office worker executes office work, an examination room (2) where a doctor and a nurse collect follicles from a patient, a test / treatment room (3) where an embryo cultivator executes various treatments to ova, and a storage room (4) where fertilized eggs are cryopreserved, **characterized in that**;
a server (13) and plural personal computers (13) are installed in the office (1), and an identification code reader (14) for the office worker is arranged in the office (1);
plural personal computers (21) are installed in the examination room (2), and an identification code reader (22) for the doctor and the nurse is arranged in the examination room (2);
a label printer (31) is installed in the test / treatment room (3), plural tablet computers (33) and plural other identification code readers (34) for the embryo cultivator are arranged in the test / treatment room (3);
a tank for cryopreservation (41) is installed in the storage room (4), ovum preservation containers (45) are stored in the tank for cryopreservation (41), and a reader (47) for the embryo cultivator capable of reading an identification code and an IC tag is arranged in the storage room (4);
an identification code (13) and an IC tag (13) are at least attached to or mounted in the ovum preservation containers (45); and
the server (13), the personal computers (13, 21), the identification code reader (14, 22, 34), the label printer (31), the tablet computers (33), the ovum preservation containers (45), and the reader (47) capable of reading an identification code and an IC tag are connected via intra-facility network (5),
wherein an inlet (51) of an IC tag (45) is contained in a cover pipe (52) mounted on an upper end of a rod-like part (45a) of the ovum preservation container (45); and
wherein the inlet (51) of the IC tag (45) is **characterized by** having a hole or a groove formed in the inlet (51) of the IC tag.

2. The individual management system for reproductive cells according to claim 1, wherein the intra-facility network (5) is further connected to a cloud server (71) via internet (6).

3. The individual management system for reproductive cells according to claim 2, wherein the cloud server (71) can be accessed by a portable information terminal device (81) of a user of the facility.

4. The individual management system for reproductive cells according to any one of claims 1 to 3, wherein the facility is a facility for reproductive cells of animals.

5. The individual management system for reproductive cells according to any one of claims 1 to 4, wherein instruments and implements used in liquid nitrogen filled up within the tank for cryopreservation (41) include at least a canister (42) and a cane (43).

6. The individual management system for reproductive cells according to claim 5, wherein an IC tag (42d) is suspended from a hooking portion (42c) of the canister (42).

7. The individual management system for reproductive cells according to claim 5, wherein an IC tag (43d) can be detachably mounted on the grasping portion (43c) of the cane (43).

8. The individual management system for reproductive cells according to any one of claims 1 to 7, wherein a container for reproductive cells further includes a sperm preservation container.

9. The individual management system for reproductive cells according to claim 8, wherein the sperm preservation container is a specimen tube (76) and an IC tag (74) is mounted in a leg portion (72a) of a body (72) of the specimen tube (76).

10. The individual management system for reproductive cells according to claim 9, wherein the sperm preservation container is a sperm preservation straw (91) and an IC tag (92) is mounted in a thin pipe (82) of the sperm preservation straw (91).

11. The individual management system for reproductive cells according to any one of claims 1 to 10, wherein an extended antenna (48) can be attached to the reader (47) capable of reading an identification code and an IC tag.

## Patentansprüche

1. Individuelles Managementsystem für Reproduktionszellen in einer Einrichtung zur In-Vitro-Befruchtung, welche ein Büro (1) enthält, wo ein Büroarbeiter Büroarbeiten ausführt, einen Untersuchungsraum (2), wo ein Arzt und eine Krankenschwester Follikel von einer Patientin sammeln, einen Test/Behandlungsraum (3), wo ein Embryokultivator an Eizellen verschiedene Behandlungen ausführt, und einen Speicherraum (3), wo befruchtete Eier cryokonserviert werden, **dadurch gekennzeichnet, dass**
ein Server (13) und mehrere Personal-Computer (13) in dem Büro (1) installiert sind, und ein Identifikationscode-Lesegerät (14) für den Büroarbeiter in dem Büro (1) angeordnet ist;
mehrere Personal-Computer (21) in dem Untersuchungsraum (2) installiert sind, und ein Identifikationscode-Lesegerät (22) für den Arzt und die Krankenschwester in dem Untersuchungsraum (2) angeordnet ist;
ein Etikettendrucker (31) in dem Test/Behandlungsraum (3) installiert ist, mehrere Tablet-Computer (33) und mehrere andere Identifikationscode-Lesegeräte (34) für den Embryokultivator in dem Test/Behandlungsraum (3) angeordnet sind;
ein Tank zur Cryokonservierung (41) in dem Speicherraum (4) installiert ist, Eizellenkonservierungsbehälter (45) in dem Tank zur Cryokonservierung (41) gespeichert sind, und ein Lesegerät (47) für den Embryokultivator, der in der Lage ist, einen Identifikationscode und ein IC-Tag zu lesen, in dem Speicherraum (4) angeordnet ist;
ein Identifikationscode (13) und ein IC-Tag (13) in dem Eizellenkonservierungsbehältern (45) zumindest angebracht oder daran montiert sind; und
der Server (13), die Personal-Computer (13, 21), das Identifikationscode-Lesegerät (14, 22, 34), der Etikettendrucker (31), die Tablet-Computer (33), die Eizellenkonservierungsbehälter (45) und das Lesegerät (47), das in der Lage ist, einen Identifikationscode und ein IC-Tag zu lesen, über ein Einrichtungs-internes Netzwerk (5) verbunden sind,
wobei ein Einlass (51) eines IC-Tags (45) in einem Abdeckrohr (52) enthalten ist, das an einem oberen Ende eines stangenartigen Teils (45a) des Eizellenkonservierungsbehälters (45) angebracht ist; und
wobei der Einlass (51) des IC-Tags (45) **dadurch gekennzeichnet ist, dass** er ein Loch oder eine Nut aufweist, das oder die in dem Einlass (51) des IC-Tags ausgebildet ist.

2. Das individuelle Managementsystem für Reproduktionszellen nach Anspruch 1, wobei das Einrichtungs-interne Netzwerk (5) ferner über das Internet (6) mit einem Cloud-Server (71) verbunden ist.

3. Das individuelle Managementsystem für Reproduktionszellen nach Anspruch 2, wobei an einem tragbaren Informationsendgerät (81) eines Benutzers der Einrichtung auf den Cloud-Server (71) zugegriffen werden kann.

4. Das individuelle Managementsystem für Reproduktionszellen nach einem der Ansprüche 1 bis 3, wobei die Einrichtung eine Einrichtung für Reproduktionszellen von Tieren ist.

5. Das individuelle Managementsystem für Reproduktionszellen nach einem der Ansprüche 1 bis 4, wobei Instrumente und Implemente, die in flüssigem Stickstoff verwendet werden, der in den Tank zur Cryokonservierung (41) gefüllt ist, zumindest einen Kanister (42) und einen Stab (43) enthalten.

6. Das individuelle Managementsystem für Reproduktionszellen nach Anspruch 5, wobei ein IC-Tag (42d) von einem Hakenabschnitt (42c) des Behälters (42) hängt.

7. Das individuelle Managementsystem für Reproduktionszellen nach Anspruch 5, wobei ein IC-Tag (43d) an dem Griffabschnitt (43c) des Stabs (43) abnehmbar angebracht werden kann.

8. Das individuelle Managementsystem für Reproduktionszellen nach einem der Ansprüche 1 bis 7, wobei ein Behälter für Reproduktionszellen ferner einen Spermakonservierungsbehälter enthält.

9. Das individuelle Managementsystem für Reproduktionszellen nach Anspruch 8, wobei der Spermakonservierungsbehälter ein Musterrohr (76) ist, und ein IC-Tag (74) an einem Beinabschnitt (72a) eines Körpers (72) des Musterrohrs (76) angebracht ist.

10. Das individuelle Managementsystem für Reproduktionszellen nach Anspruch 9, wobei der Spermakonservierungsbehälter ein Spermakonservierungshalm (91) ist, und ein IC-Tag (92) in einem dünnen Rohr (82) des Spermakonservierungshalms (91) angebracht ist.

11. Das individuelle Managementsystem für Reproduktionszellen nach einem der Ansprüche 1 bis 10, wobei eine verlängerte Antenne (48) an dem Lesegerät (47) angebracht werden kann, das in der Lage ist, einen Identifikationscode und ein IC-Tag zu lesen.

## Revendications

1. Un système de gestion individuelle pour les cellules reproductrices dans un centre de fécondation in vitro qui comprend un bureau (1) où un employé de bureau exécute du travail de bureau, une salle d'examen (2) où un médecin et une infirmière recueillent les follicules d'une patiente, une salle de test/traitement (3) où un cultivateur d'embryons exécute divers traitements sur les ovules, et une salle de stockage (4) où les ovules fécondés sont cryoconservés, **caractérisée en ce que** :
un serveur (13) et plusieurs ordinateurs personnels (13) sont installés dans le bureau (1) , et un lecteur de code d'identification (14) pour l'employé de bureau est disposé dans le bureau (1) ;
plusieurs ordinateurs personnels (21) sont installés dans la salle d'examen (2), et un lecteur de code d'identification (22) pour le médecin et l'infirmière est installé dans la salle d'examen (2) ;
une imprimante d'étiquettes (31) est installée dans la salle de test/traitement (3), plusieurs ordinateurs tablettes (33) et plusieurs autres lecteurs de codes d'identification (34) pour le cultivateur d'embryons sont disposés dans la salle de test/traitement (3) ;
un réservoir pour la cryoconservation (41) est installé dans la salle de stockage (4), des conteneurs de conservation des ovules (45) sont stockés dans le réservoir pour la cryoconservation (41), et un lecteur (47) pour le cultivateur d'embryons capable de lire un code d'identification et une étiquette IC est installé dans la salle de stockage (4) ;
un code d'identification (13) et une étiquette IC (13) sont au moins attachés ou montés dans les conteneurs de conservation des ovules (45) ; et
le serveur (13), les ordinateurs personnels (13, 21), le lecteur de code d'identification (14, 22, 34), l'imprimante d'étiquettes (31), les tablettes électroniques (33), les conteneurs de conservation des ovules (45) et le lecteur (47) capable de lire un code d'identification et une étiquette IC sont connectés via le réseau du centre interne (5),
dans lequel une entrée (51) d'une étiquette IC (45) est contenue dans un tuyau de couverture (52) monté sur une extrémité supérieure d'une partie en forme de tige (45a) du conteneur de conservation des ovules (45) ; et
dans lequel l'entrée (51) de l'étiquette IC (45) est caractérisé en ayant un trou ou une rainure formé dans l'entrée (51) de l'étiquette IC.

2. Le système de gestion individuelle pour les cellules reproductrices selon la revendication 1, dans lequel le réseau du centre interne (5) est en outre connecté à un serveur cloud (71) via Internet (6).

3. Le système de gestion individuelle pour les cellules reproductrices selon la revendication 2, dans lequel le serveur cloud (71) peut être consulté par un terminal d'information portable (81) d'un utilisateur du centre.

4. Le système de gestion individuelle des cellules reproductrices selon l'une des revendications 1 à 3, dans lequel le centre est un centre pour les cellules reproductrices d'animaux.

5. Le système de gestion individuelle des cellules reproductrices selon l'une quelconque des revendications 1 à 4, dans lequel les instruments et outils utilisés dans l'azote liquide rempli dans le réservoir pour la cryoconservation (41) comprennent au moins une boîte (42) et une canne (43).

6. Le système de gestion individuelle des cellules reproductrices selon la revendication 5, dans lequel une étiquette IC (42d) est suspendue à une partie d'accrochage (42c) de la boîte (42).

7. Le système de gestion individuelle pour les cellules reproductrices selon la revendication 5, dans lequel une étiquette IC (43d) peut être montée de manière amovible sur la partie de préhension (43c) de la canne (43).

8. Le système de gestion individuelle des cellules reproductrices selon l'une des revendications 1 à 7, dans lequel un conteneur de cellules reproductrices comprend en outre un conteneur de conservation du sperme.

9. Le système de gestion individuelle des cellules reproductrices selon la revendication 8, dans lequel le récipient de conservation du sperme est un tube à spécimen (76) et une étiquette IC (74) est montée dans une partie de jambe (72a) d'un corps (72) du tube à spécimen (76).

10. Le système de gestion individuelle des cellules reproductrices selon la revendication 9, dans lequel le récipient de conservation du sperme est une paille de conservation du sperme (91) et une étiquette IC (92) est montée dans un tuyau fin (82) de la paille de conservation du sperme (91).

11. Le système de gestion individuelle des cellules reproductrices selon l'une des revendications 1 à 10, dans lequel une antenne étendue (48) peut être attachée au lecteur (47) capable de lire un code d'identification et une étiquette IC.
